# EUROPEAN PATENT SPECIFICATION

(11) **EP 1 773 721 B1**
(45) Date of publication and mention of the grant of the patent: **05.10.2011**
(21) Application number: 05761486.9
(22) Date of filing: 10.06.2005
(51) Int. Cl.: C02F 1/44, C02F 3/34

(54) **PROCESS FOR RECOVERING THE COMPONENTS OF OLIVE MILL WASTEWATER WITH MEMBRANE TECHNOLOGIES**
VERFAHREN ZUR RÜCKGEWINNUNG DER BESTANDTEILE DES ABWASSERS EINER OLIVENMÜHLE DURCH MEMBRANTECHNOLOGIE
PROCEDE DE RECUPERATION DE COMPOSES DE RESIDUS AQUEUX DE MOULINS A HUILE D'OLIVE UTILISANT DES TECHNIQUES MEMBRANAIRES

(30) Priority: 16.06.2004 IT RM20040292
(43) Date of publication of application: 18.04.2007
(73) Proprietor: Enea-Ente Per Le Nuove Tecnologie, L'Energia e L'Ambiente, 00196 Roma (IT); Verdiana S.R.L., 89022 Cittanova RC (IT)
(72) Inventor: PIZZICHINI, Massimo ENEA, I-00196 Roma (IT); RUSSO, Claudio ENEA, I-00196 Roma (IT)
(74) Representative: Banchetti, Marina
(86) International application number: PCT/IT2005/000327
(87) International publication number: WO 2005/123603

(56) References cited:
- IT-B- 1 204 691
- US-A1- 2002 096 473
- TURANO E ET AL: "An integrated centrifugation-ultrafiltration system in the treatment of olive mill wastewater" JOURNAL OF MEMBRANE SCIENCE, ELSEVIER SCIENTIFIC PUBL.COMPANY. AMSTERDAM, NL, vol. 209, no. 2, 15 November 2002 (2002-11-15), pages 519-531, XP004388861 ISSN: 0376-7388 cited in the application
- CHERYAN M ET AL: "Membrane processing of oily streams. Wastewater treatment and waste reduction" JOURNAL OF MEMBRANE SCIENCE, ELSEVIER SCIENCE, AMSTERDAM, NL, vol. 151, no. 1, 9 December 1998 (1998-12-09), pages 13-28, XP004152780 ISSN: 0376-7388

## Description

The present invention concerns a process for totally recovering the chemical components of olive mill wastewater with membrane technologies. More specifically, the invention concerns a selective fractioning process of the components present in olive mill wastewater, enabling recovery of the polyphenolic compounds contained therein, the reuse of the concentrate residues for the production of fertilizers and possibly biogas, and the obtaining of a highly purified aqueous product which can also be used as a basic component for beverages.

As is known, olive oil production generates considerable amounts of wastewater (i.e., the olive mill wastewater, OMW) every year, as well as great amounts of solid waste - the olive residues - whose disposal with respect to current legislation is one of the biggest environmental problems of the Mediterranean region. The quantity of olive mill wastewater normally generated in relation to the processed product depends on the type of olive processed, the extraction system used and the processing conditions, but it may be considered that wastewater makes up an average 70-120% of the weight of processed olives. The polluting impact of this wastewater, expressed in Chemical Oxygen Demand (COD), is estimated on average between 40 and 195 g/I of oxygen, with a BOD (Biological Oxygen Demand) of about 35-100 g/l of O₂.

In general, the olives intended for the production of extra-virgin olive oil are first sorted to eliminate the leaves, washed in water, crushed using various techniques (pan mills and other forms of crushers and presses based on hammers, discs, rollers, cutters or mixed types), and then undergo kneading, which serves to remix the olive paste to facilitate the aggregation of the micro-drops of oil, which are separated out from the water and from the solid components through mechanical means. This mechanical separation of the oil, which may be obtained by pressing or centrifugation, yields the oil itself, wastewater and olive residues.

Two different extraction systems are used in olive oil production: the traditional olive mill, which implements a discontinuous process of pressing, and the continuous system, which works by centrifugation. Both systems envisage an initial processing stage wherein the olives are washed and then reduced to a paste inside a mechanical crusher. The paste then undergoes pressing, in the case of traditional olive mills, with the production of a solid residue - the olive residues - and a liquid product called "must". The "must" is then centrifuged to separate out an oily fraction that constitutes the oil of first pressing, while the aqueous fraction is the wastewater of this process. As regards the continuous type systems, called "three-phase systems", which are more widespread today, the olive paste is sent directly to separation by centrifugation, after being appropriately diluted with hot water. This operation yields three phases: the oil of first pressing, the olive reidues and the wastewater. It is obvious that, in this case, the aqueous solution obtained as residue is, with the same amount of olives processed, quantitatively greater, albeit with a lower content of dissolved solids.

Over the last few years, the traditional olive oil producing countries have seen a considerable development in the extra-virgin olive oil mechanical extraction process, with a gradual shift from the original discontinuous pressing process to the more productive continuous ones based on horizontal centrifuges (decanters). The shift from a discontinuous process to a three-phase continuous one has considerably reduced labour and improved hygiene but, as already mentioned, it has also increased the production of wastewater. Moreover, the water contents of the olive residues has also increased with this process, going from 24-30% to 48-54% approx.

The need to improve olive oil quality and to reduce the environmental impact of the wastewater has led to considering the possibility of modifying the three-phase decanter extraction process by reducing the amount of process water added. This has led to developing the so-called "two-phase" centrifugation extraction systems, wherein water is not added to the decanter and the by-product obtained consists of a single phase made up of a mixture of solid residues and olive mill wastewater.

OMW disposal is a big problem for the olive oil industry, because of the great environmental impact of this waste as such and the difficulty of recovering any commercially useful substances from it. In general, olive mill wastewater is a dark cloudy strong-smelling liquid that is easily fermentable. It is rich in organic substances, given the fact that it contains the water making up the olives, with a vast series of organic and inorganic compounds in solution and suspension, as well as the water used for washing the machines themselves and - in the case of three-phase continuous systems, as already mentioned - there is also a considerable amount of water used for diluting the olive paste. Owing to the high concentration of organic substances and to their nature, which is hardly biodegradable and in some cases even biotoxic, as well as their acidity, the direct disposal of the OMW in the sewage system is not allowed. Firstly, because of its high organic acid content, this wastewater would corrode piping and, secondly, the suspended solids contained (4-17% on average) could block the pipes near the olive mill and thus impede then normal circulation of waste. Moreover, if the sediment is disposed of directly in sewers, it could start an anaerobic fermentation process with the resulting foul odours and increase in acidity.

Since the polluting impact of OMW is, in varying degrees of magnitude, greater than that of urban wastewaters (the polluting impact of 1 m³ of olive mill wastewater is approximately equivalent to 100-200 m³ of domestic wastewaters), its direct treatment in the usual urban sewage processing plants is unthinkable, above all, for its high content of hardly biodegradable and bacteriostatic substances, which interfere with the actual functioning of the biological sewage treatment plants. Moreover, the problem is considerably stressed by the seasonal nature of olive mill wastewater production which, at least for the Mediterranean region, accumulates in large volumes in just the November-January period.

In addition, there is also the problem of the olive residues, which no longer have a market for olive residues oil production. These organic waste products are thus another waste disposal problem for olive mills, and their disposal through burning is inconvenient due to the low heat value of the raw material and the foul odours of the gases given off.

More specifically, despite the variations of concentration and composition depending on the areas of production, the climatic conditions during olive ripening, the harvesting practices, the oil extraction process and so on, it may be said that the OMW contains from 5 to 25% in weight of dissolved or suspended substances, mostly composed of organic material and with a lesser degree of inorganic compounds. The organic substances are mostly sugars, which makes this wastewater easily subject to fermentation; and also polyphenol compounds, which are mainly responsible for its biotoxicity; organic acids, which determine its weakly acid pH; proteins and azotic compounds in general, fatty substances, mixed phenol-polysaccharide polymers, polyalcohols, cellulose and hemicellulose, pectins and tannins. The inorganic substance includes mainly potassium and to a lesser degree sodium, calcium and magnesium as cations, and chlorides, phosphates and sulphates as anions.

As already observed, one component that makes olive mill wastewater particularly harmful if dispersed unprocessed, such as by directly pouring it into soils as a fertilizer (something that many oil producers still do) is its content of polyphenol compounds. These are natural components of OMW, which act in the olives as protective agents with antioxidant and bacteriostatic functions until the olives ripen. Conversely, the said polyphenols also have a phytotoxic action (within certain limits) and are harmful to the bacterial flora in the soil, so that the uncontrolled use of the olive mill wastewater as a fertilizer can, in particular cases, damage crops and the quality of underground water sources. Small quantities of polyphenols can be metabolised by soil microbes, even with a certain level of fertilization, but the doses employed must never be excessive. In particular, current legislation in Italy establishes the maximum doses at 50 m³/ha for wastewater coming from traditional discontinuous olive mills and 80 m³/ha for wastewater coming from continuous processes. Moreover, land closer than 300 m to safeguarded water collection areas for public consumption and for current horticultural crop use are excluded from this practice, as well as areas with a water table of less than 10 m depth and, finally, snow-covered or water saturated land.

The negative effects of directly pouring olive mill wastewater into soils has also been seen on soil properties, such as the immobilisation of the available nitrogen, the formation of amphysols by replacement of potassium in calcium complexes, the increase in salinity and the reduction of magnesium available for plants, probably owing to the antagonistic effect of potassium.

Still owing to the presence of polyphenolic compounds in OMW, other conventional methods such as storage in evaporation tanks with the production of partly oxidised sludge does not completely solve the environmental problem because the sludge ends up on the soil. Moreover, the same legislation established that wastewater storage at olive mills must not exceed 30 days - a limit later extended to 3 months.

The OMW disposal problem has long been the subject of scientific research and experimental applications, and many systems have been proposed in order to avoid a direct and uncontrolled disposal of this by-product in the environment. As noted, the problem is stressed by the fact that olive oil production is normally spread over a large number of small scale, often rural, producers with limited access to of industrial technologies and limited availability of qualified personnel for running sophisticated equipment, and by the fact that it is a seasonal production.

Among the solutions put forward for OMW disposal, some of which are still at an experimental stage, it may be firstly recalled single- or multiple-effect evaporation under vacuum, with the production of a concentrated and more easily disposable waste material, which can be reused in the production of animal feed or as fertilizer, and an aqueous condensate. In order for the latter to be disposed of as a liquid effluent, according to current environmental legislation, it must in any case be further purified, such as via biological treatment. Moreover, the considerable energy consumption makes this type of systems viable only for large scale industrial olive mills.

A great many chemical-physical treatments have also been proposed, such as separating the solid particle components by filtering or centrifugation, or coagulation by adding appropriate coagulating agents, and flocculation - normally carried out by adding polyelectrolyte flocculants. These technologies evidently allow achieving a limited level of depuration and in any case produce residues that cannot be directly released into the environment. Thus, in this case, too, the treatment must be combined with further purification phases.

Other treatment systems proposed use various biological techniques - either singly or in a combination with other methods - in order to reduce the polluting impact of olive mill wastewater. Both aerobic and anaerobic treatments have been experimented, which enable a great reduction of the COD and BOD of the wastewater. The relative equipment is difficult to handle and to start up seasonally. In particular, the anaerobic treatment plants, which have the advantage of being much more compact than aerobic ones and of producing lower amounts of solid waste (to be used as fertilizers) and, above all, of biogas (methane), have the problem of requiring some months for start-up and thus are not suitable for treating the seasonal wastes of olive oil production. These plants colud be used by other industrial plants mixing their wastes with those of olive oil production.

Various attempts at turning OMW into a fertilizing substrate have been made, demonstrating the fertilizing properties of the treated wastewater and minimising the toxic effects on soils. Moreover, various compost production processes for obtaining fertilizers have been studied starting from olive mill wastewater and spent olive residues. By using highly absorbent solid organic matrices, such as triturated hay, olive leaves or sawdust, as well as the spent olive residues and fractions of their separation process, like powder and peel, and by absorbing the wastewater on these materials (normally by adding suitable agents), it is possible to create a compost production process for turning - over time - the waste products into compost for use as an organic fertilizer. It is obvious, however, that such a system can enable the disposal of limited quantities of OMW, because of its great dilution. Moreover, even this system has the same limitation of not being able to completely eliminate the biotoxicity of some components present in the wastewater, which interfere with the aerobic oxidative process of compost production.

Anaerobic fermentation processes envisaging a pre-treatment phase using lime have also been studied, and these have provided some interesting results both in terms of COD abatement (91 %), and as regards methane production (0.80 g CH₄/g total of COD). In this case, as already observed, the problem lies in the slow biochemistry of the anaerobic process, coupled to the fact that OMW is only available 3-4 months a year.

Electrochemical systems have also been proposed for oxidising, in reactors, all the polyphenolic components of the wastewater, but the energy costs are too high.

Still with the aim of reducing the organic component of OMW in order to be able to release it, purified, into the environment, membrane separation processes have already been proposed, such as ultrafiltration and reverse osmosis. For example, a depuration treatment has been proposed, wherein the olive mill wastewater first undergoes a preliminary sedimentation phase to allow the residual oil droplets and part of the suspended solid particles to separate out from the wastewater itself, and is then directly subjected to ultrafiltration on polymeric membranes (R. Borsani, B. Ferrando, Ultrafiltration plant for olive vegetation waters by polymeric membrane batteries, Desalination, 108, pp. 281-286, 1996). The permeated liquid, which still has a COD above 30,000 ppm of O₂, then undergoes biological depuration treatment together with urban wastewaters.

It is evident, however, that in order to be treated by using semipermeable membranes, the OMW must undergo preliminary treatment, because its high concentration of suspended solids and colloidal materials hinders its passage through the membrane. Another treatment method that uses membrane technologies envisages the preventive separation of the suspended solids by means of centrifugation, followed by ultrafiltration treatment of the supernatant obtained from the centrifugation (E. Turano, S. Curcio, M. G. De Paola et al., An integrated centrifugation-ultrafiltration system in the treatment of olive mill wastewater, J. of Membrane Science, 209, pp. 519-531, 2002). The ultrafiltration permeate thus obtained achieves a COD abatement of about 90%. However, this value (about 5,000 mg of O₂/l) is still too high to allow its direct disposal in sewage systems according to current European environmental laws.

According to another process, proposed in general for the treatment of agrofood industry liquid waste, including liquids containing animal excrements, wine industry washing waters and olive mill wastewater, described in the international patent application published with No. WO 96/09986 (Rhône-Poulenc Chimie), the wastewater can be preliminarily treated with a liquid-solid separation operation (consisting of centrifugation, decanting or pressing), followed by a chemical-physical coagulation-flocculation treatment which provides a liquid phase with a reduced amount of solids in suspension. According to the proposed process, the liquid phase resulting from the previous treatments first undergoes either ultra- or microfiltration and then a final-treatment of reverse osmosis on the permeate of the preceding filtration. According to this document, the reverse osmosis yields a liquid with a sufficiently low COD to allow its disposal directly in the environment, while the retentate streams either of the ultra- or microfiltration or of the reverse osmosis are fed back to the initial solid-liquid separation phase. All the solid materials obtained in the process are destined for compost production. The described process does not seem to have been applied specifically to olive mill wastewater, so it is not ascertained whether it enables a COD abatement according to current legislation (Italian legislative decree No. 152/99) for this particular type of wastewater. Moreover, the destination of solid residues to compost production has the same inconveniences already mentioned above, due to the presence of bacteriostatic polyphenol compounds in the OMW.

The US patent application published under No. US 2002/0096473 (Ferro et al.) discloses a method for treating potato wastewater comprising the steps of (a) pretreating a potato wastewater stream including suspended and dissolved solids with an alpha amylase enzyme to hydrolyze starch in the wastewater, (b) ultra-filtering the enzyme-treated wastewater from step (a) to filter and remove a portion of the suspended solids in a first concentrate stream separated from the wastewater, and (c) reverse osmosis-filtering the ultra-filtered wastewater from step (b) to remove at least about 70% of the wastewater dissolved and suspended solids in a second concentrate stream.

More recently there has been more in-depth study of aspects linked to the biomedical properties of the polyphenol families present in OMW (G. F. Montedoro, M. Servili, Recupero di polifenoli dalle acque di vegetazione delle olive e valutazione del loro potere antiossidante, Industrie Alimentari, 28, 14-19, 1989; M. Servili et al., HPLC evaluation of phenols in olive fruit, virgin olive oil, vegetation waters and pomace, and 1 D- and 2D-NMR characterization, J. Am. Oil Chem. Soc., 76, pp. 873-882, 1999), highlighting their considerable antioxidant properties and their beneficial effects in maintaining the organoleptic properties of olive oil (preventing rancidity) and for the nutraceutic value of the product containing them. Various laboratory studies have reported that the most important product of hydrolysis of oleuropein (a glucoside present in olive oil); hydroxytyrosol [(3,4-dihydroxyphenyl)ethanol] has a marked chemo-protective effect (C. Manna et al., Biological effects of hydroxytyrosol, a polyphenol from olive oil endowed with antioxidant activity, Adv. Exp. Med. Biol., 472, pp. 115-130, 1999) and, in particular, inhibits oxidation of the LDL, a process involved in the pathogenesis of atherosclerosis (O. I. Auroma et al., Effects of hydroxytyrosol found in extra virgin olive oil on oxidative DNA damage and on low-density lipoprotein oxidation, J. Agric. Food Chem., 46, pp. 5181-5187, 1998), inhibits platelet aggregation (F. Visioli et al., Antioxidant and other biological activities of olive oil mill waste water, J. Agric.Food Chem., 47, pp. 3397-3401, 1999) and presents an in-vitro antibacterial activity (A. Bisignano et al., On the in-vitro antimicrobial activity of oleuropein and hydroxytyrosol, J. Pharm. Pharmacol., 51, pp. 971-974, 1999).

The aforesaid chemical characterisation, biological and pharmacological studies were carried out on small quantities of solution and did not involve a specific treatment process for the enormous amounts of wastewater generated by olive mills. However, they do clearly indicate a remunerative possibility for OMW treatment process, that is, the valorisation and marketing of antioxidant molecules of the polyphenol type that can be separated out from this wastewater.

In the light of the above, the present invention aims to provide an olive mill wastewater treatment process that is efficient and easy to implement from the installation and management standpoint, so that is can be used within the olive mill itself - thus avoiding the transportation of huge volumes of wastewater and spent olive residues - while, at the same time, enabling the recovering of secondary raw materials of commercial value, so that the revenues deriving from the recovery of this refined material can compensate for the costs of the depuration treatment itself.

To this end, and on the basis of the typical chemical composition of olive mill wastewater as well as the aforesaid strategic aims, the present invention firstly proposes to appropriately control the milling process and the phases immediately following it in order to obtain the maximum amount of commercially useful polyphenolic compounds in the wastewater before subjecting it to the depuration process. To this end, according to the present invention, it is considered that the polyphenolic compounds and the glucosides initially containing them (secoiridoids, of which oleuropein is the most important member), which are present in the highest concentration in the ripe olive, are gradually degraded by oxidant enzymes as the olive ages, and as the OMW is left idle after milling. That is why, according to the present invention, the OMW must be collected as soon as possible in order for it to undergo the depuration treatment and to recover the useful substances in good time.

The pH of the freshly produced olive mill wastewater is first adjusted to a value within 3 and 4.5, with the triple aim of: deactivating the polyphenol-oxidase enzyme, which would otherwise tend to oxidise the useful polyphenolic compounds; favouring the transformation of oleuropein into hydroxytyrosol, which tends to take place spontaneously in an acid pH; creating the optimal conditions for subsequent enzymatic treatment. The wastewater is then, according to the present invention, subjected to enzymatic hydrolysis in order to degrade and separate out the cellulose, hemicellulose and pectin microdispersed components from the wastewater, so that the degradation products can be separated via centrifugation in order to obtain a partially clarified liquid fraction.

Still according to the present invention, the liquid stream obtained by centrifugation is subjected to tangential microfiltration (MF), carried out in "back washing" hydraulic conditions, preferably followed by tangential ultrafiltration (UF), carried out on the permeate of the microfiltration. Both the MF and UF operations are followed by the diafiltration (DF) technique, which enables the maximisation of the recovery of polyphenolic components in the respective permeates. The concentrated effluents of DF - both from the MF and from the UF - are added to the final solid residue of the centrifugation and can be advantageously used - possibly together with the spent olive residues - as a basis for compost production or aerobic treatment, or even for anaerobic treatment. Since these liquids have been depleted of their polyphenolic compounds, which thanks to the treatments carried out are now highly concentrated in the MF (and then UF) permeate streams, the inconvenience due to the biotoxicity of these compounds in biological treatments and in compost production will be attenuated by using the procedure according to the following invention.

The permeate coming from MF (or preferably from the subsequent UF phase), to which the permeates from DF have been added, is - still according to the present invention - subjected to a tangential nanofiltration (NF) treatment, in order to recover the active polyphenol molecules mostly in the permeate. The concentrate obtained from NF is also subjected to the DF technique and the DF permeate then undergoes reverse osmosis (RO).

After the diafiltration, the NF concentrate contains various polyphenol families, including hydroxytyrosol, caffeic acid, coumaric acid, tyrosol, etc. These compounds can find useful applications as antioxidants in the agrofood industry, in cosmetics and also in the functional food sector.

The NF permeate, which contains the maximum amount of hydroxytyrosol and other polyphenols of biomedical interest, is subjected to a final membrane treatment - reverse osmosis (RO). This process yields a concentrate liquid rich in active ingredients, and a permeate made up of extremely purified water, because it is practically devoid of mineral salts and organic substances, but still contains a certain amount of active polyphenol compounds that make it suitable for producing special beverages.

The treatment proposed according to the present invention not only provides for abating the polluting impact of olive mill wastewater, so that the latter can be directly disposed of in the environment with full compliance of current legislation, but also uses this OMW as a raw material for obtaining the following commercially useful products: organic fertilizers, polyphenol-based antioxidants, and ultrapure water that can be used as a base for producing hypotonic beverages or beverages of a biomedical interest.

Therefore, the present invention specifically concerns a process for fully recovering the chemical components of olive mill wastewater (OMW) including, in sequence, the following operations:
a) collecting the raw OMW after olive milling;
b) adjusting the pH of the said OMW to within the range of 3-4.5;
c) treating the said wastewater obtained in the preceding operation to enzymatic hydrolysis;
d) separating the wastewater resulting from the preceding operation into its constituent particle and suspended components, on the one hand, and a partially clarified liquid product, on the other;
e) treating the said liquid resulting from operation d) by tangential microfiltration (MF), and providing a retentate phase and a permeate phase;
f) treating the permeate deriving from the preceding operation to tangential nanofiltration (NF), and providing a retentate phase and a permeate phase;
g) treating the permeate deriving from operation f) to reverse osmosis (RO), and providing a retentate phase rich in purified polyphenols and a permeate composed of purified water;
the said solid phase resulting from operation d) and the said retentate phase resulting from operation e) being reusable as a substrate for compost production or for anaerobic fermentation processes, and the said retentate phase resulting from operation f) being reusable for extracting polyphenol compounds therefrom.

Preferably, after the said operation e) of tangential microfiltration (MF) the following further operation is carried out:
e1) treating the said retentate phase obtained from MF by adding water and carrying out another microfiltration (diafiltration), to increase polyphenol extraction in the permeate product;
the liquid subjected to the subsequent operation f) of nanofiltration being the sum of the permeate and of the diafiltrate coming from the preceding operaton.

Preferably, after microfiltration and before nanofiltration, on the permeate line, the process also includes the following operation:
e2)treatment by tangential ultrafiltration (UF) in order to obtain a retentate phase which can be reused as a substrate for compost production or for aerobic or anaerobic fermentation processes and a permeate which is fed into the next operation f) of nanofiltration.

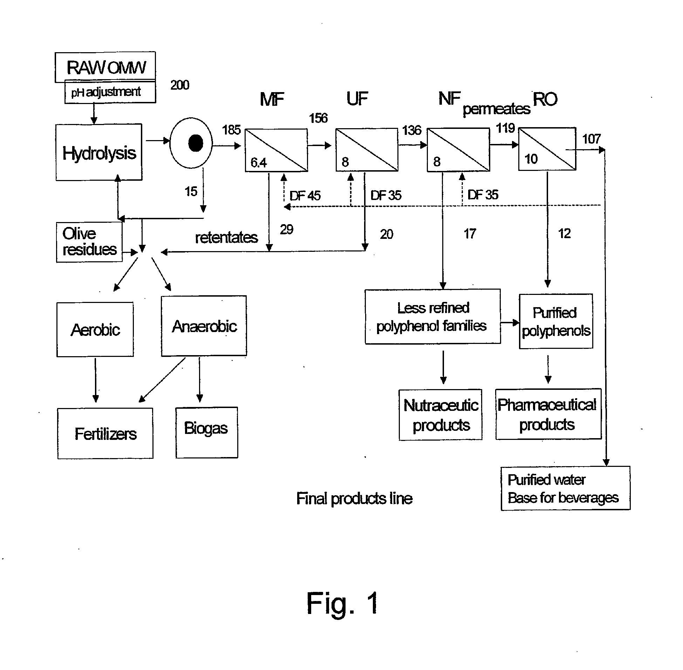

The specific features of the invention, and particularly the relative operative procedures of the proposed depuration and recovery process, will be clearer with reference to a blocke diagram illustrated in Figure 1, which summarises both a preferred embodiment of the process of the present invention and the possible destinations and uses of the products obtained therefrom.

According to the currently preferred versions of the present invention, the phase a) of OMW collection is carried out between 1 and 3 hours, at the most, after the wastewater has been generated in the olive mill, in order to avoid an excessive oxidation of the useful polyphenol compounds, which must be recovered from the liquid. In operation b) the wastewater pH is adjusted to within the range 3-4.5, preferably by adding hydrogen chloride and citric acid, while operation c) of enzymatic hydrolysis is carried out by adding a pectinase enzymatic complex to the acidified wastewater. According to the currently preferred embodiment of the present invention, the said pectinase enzymatic complex is a pectinase enzymatic complex extracted from *Aspergillus niger,* specifically Pectinex SMASH XXL (Novo Nordisk), and it is left to react on the OMW at room temperature, or at 30-45°C at the most, for 3-5 hours.

The operation d) of separation is carried out advantageously by continuous centrifugation, with the production of a partially clarified liquid supernatant - which then undergoes the subsequent operation e) - and of a dense deposit that is wholly or partly fed back into operation c) of enzymatic hydrolysis. The final solid residue, or part of it, can be reused as a substrate for compost production or for anaerobic fermentation processes.

Still according to the present invention, the operation e) of tangential microfiltration is carried out with ceramic membranes of molecular size ranging between 0.10 and 1.4 µm, preferably made up of a ceramic block with 23 filtration channels in a daisy-like or sunflower-like arrangement, and each with an average diameter of 3.4 mm and length of 1.2 m, with a filtering surface of 0.35 m² per ceramic block.

The operation e2) of tangential ultrafiltration is preferably carried out with polymeric membranes of molecular size ranging between 1 and 20 kDalton, and spiral-shaped, made of one of the following materials: polysulphone, polyethersulphone polyamide or regenerated cellulose acetate. Preferably, the filtering ducts have the following sizes: 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

As more clearly illustrated in figure 1, both downstream of MF operation e) and upstream of UF operation e1) a dialfiltration operation is carried out, by feeding the relative membranes with purified water, preferably obtained from the operation g) of reverse osmosis, which is added to the retained effluent of the respective MF and UF membranes.

Still according to the present invention, operation f) of tangential nanofiltration is carried out with polymeric membranes of molecular size ranging between 150 and 250 Dalton, preferably of about 200 Dalton, spiral-shaped and made of composite polyamide or nylon. The operation g) of reverse osmosis is carried out with high saline rejection spiral-shaped polymeric membranes made of composite polyamide. Both for NF and RO, the filtering ducts may have the following sizes: 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

By applying the described process, the purified water product obtained as a permeate of operation g) of reverse osmosis has a COD lower than 100 ppm of O₂, and a TOC (Total Organic Carbon) of about 15 ppm.

Some experimental results obtained by applying one specific embodiment of the process according to the present invention are described for merely illustrative purposes in the following examples and are also illustrated in the attached drawings, wherein:
Figure 1, already commented, shows a block diagram of a process for totally recovering the chemical components of olive mill wastewater according to the present invention;
Figure 2 shows the trend over time of the flow of permeate through the microfiltration membrane described in the Example;
Figure 3 shows the trend over time of the flow of permeate through the ultrafiltration membrane described in the Example;
Figure 4 shows the trend over time of the flow of permeate through the nanofiltration membrane described in the Example; and
Figure 5 shows the trend over time of the flow of permeate through the reverse osmosis membrane described in the Example

### EXAMPLE

### 1. Wastewater collection

200 l of wastewater is collected from an olive mill using a continuous three-phase process. One hour after the OMW has been generated in the olive mill, it is subjected to a series of treatments according to the present invention, as described below.

### 2. pH adjustment

To avoid oxidation of the polyphenols contained in the OMW, its pH value is lowered from the original value of 5.7 to 3.5 in order to deactivate the polyphenol-oxidase enzyme present in the raw OMW and to create the optimum conditions for the pectinase enzyme that is added afterwards. The pH is lowered by adding concentrated HCl and citric acid (1% P/V), which serves to protect polyphenol oxidation.

The operation is checked by using a laboratory pHmeter equipped with a glass electrode.

### 3. Enzymatic treatment

To reduce the clogging effect on the membranes by the solids present in the OMW matrix, a commercially produced enzymatic complex is used, manufactured by the Novo Nordisk company and called Pectinex SMASH XXL extracted from *Aspergillus niger.*

About 20 ml of Pectinex is added to 200 l of solution taken from the previously described treatment, and the solution is left to react for about 5.0 hours under mild stirring, at a temperature of 40°C. A stratification is seen at the end of the reaction, with a suspended supernatant and an underlying partially clarified solution. The whole solution is subjected to centrifugation.

### 4. Centrifugation

The centrifugation operation is carried out in batches, at a rotation speed of 4,300 revs per minute. At the end, after 15 minutes, three layers of material have been separated: a superficial film, made up of an oily emulsion, an intermediate rather opaque solution and a dark deposit that stays at the bottom of the test tube.

The operation may be carried out by continuous centrifugation, preferably at 5,000 revs per minute, obtaining a partially clarified liquid that is then subjected to the subsequent membrane operations and a solid residue which may be fed back into the hydrolysis phase.

By centrifuging in batches, the initial 200 l yield about 185 l of permeate and 15 l of dark sediment, which is then sent to the compost production line along with the olive residues.

### 5. The microfiltration operation

The solution made up of about 185 l is filtered by means of a tangential microfiltration technology, using a Tami sunflower-shaped ceramic block membrane with a filtering surface of 0.35 m².

The process conditions are the following:
■ Feed flow rate: 4,000 l/h
■ Entry flow pressure: 2.5 bar
■ Exit flow pressure: 2.3 bar
■ Pressure on permeated side: 0.7 bar
■ Temperature: 25-30°C
■ Flow rate of the fluid inside the membrane ducts: 6 m/second.

During the trial, the average flow of permeate is around 65 l/m²h. Starting from 185 l of liquid feed, 29 l of concentrate and 156 l of permeate are collected. The MF plant contains a water-based heat-exchange system that stabilises the temperature in the range indicated.

After the concentration operation, dialfiltration is carried out by adding 35 l of distilled water to the MF concentrate, and the MF procedure is carried out again in order to obtain another 35 l of permeate. This operation serves to further extract the remaining polyphenolic components from the concentrate.

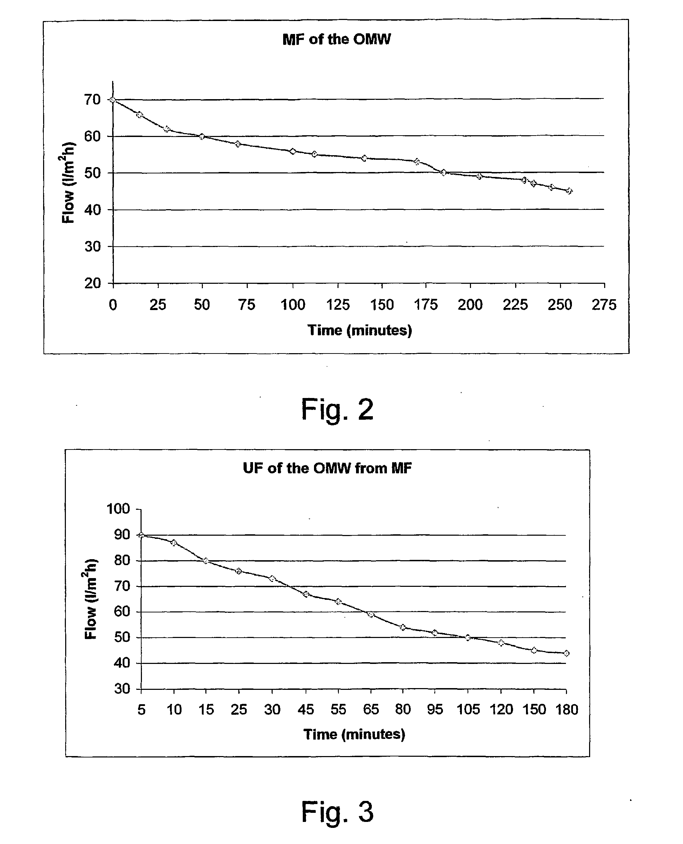

The diagram of Figure 2 shows the permeate flow trend over time (membrane productivity) at a VCR of about 6.4.

### 6. The ultrafiltration operation

The previous MF permeate is filtered by means of the ultrafiltration technique by using a spiral-shaped membrane manufactured by MICRODYN - NADIR GmbH, type Spiracel WS P005 4040 C, with a molecular size of 5 kdalton, in regenerated cellulose acetate, with a mesh spacer of 45 mil inch.

The process conditions are the following:
■ Feed flow rate: 4,000 m/h
■ Entry flow pressure: 3-4 bar
■ Exit flow pressure: 2.8-3.9 bar
■ Pressure on permeated side: zero
■ Temperature: 15-25°C
■ Flow rate of the fluid on the membrane ducts: 1.4 m/second.

The UF plant contains a water-based heat-exchange system that stabilises the temperature in the range indicated. During the trial, the average flow of permeate is around 50-60 l/m²h. Starting from 156 l of liquid feed, 20 l of concentrate and 136 l of UF permeate are collected.

After the concentration operation, dialfiltration is carried out by adding 25 l of distilled water to the UF concentrate, and the UF procedure is carried out again in order to obtain another 25 l of permeate. This operation serves to further extract the remaining polyphenolic components from the concentrate.

The diagram of Figure 3 shows the permeate flow trend over time (membrane productivity) at the operating conditions described.

The concentrate may be sent to a compost production line or to an aerobic line for the production of methane and fertilizer. The permeate is further treated by means of nanofiltration.

### 7. The nanofiltration operation

The previous UF permeate was subjected to a tangential flow nanofiltration treatment, using a spiral-shaped membrane manufactured by Osmonics (USA), type DK 4040 F1020, of composite polyamide material.

The process conditions are the following:
■ Feed flow rate: 4,000 l/h
■ Entry flow pressure: 12 bar
■ Exit flow pressure: 10 bar
■ Temperature: 20-25°C
■ Flow rate of the fluid on the membrane: 1.4 m/second.

The NF plant, which is the same as the UF plant, contains a water-based heat-exchange system that stabilises the temperature in the range indicated. During the trial, the average flow of permeate is around 35 l/m²h. Starting from 136 l of liquid feed, 17 l of concentrate and 119 l of permeate are collected.

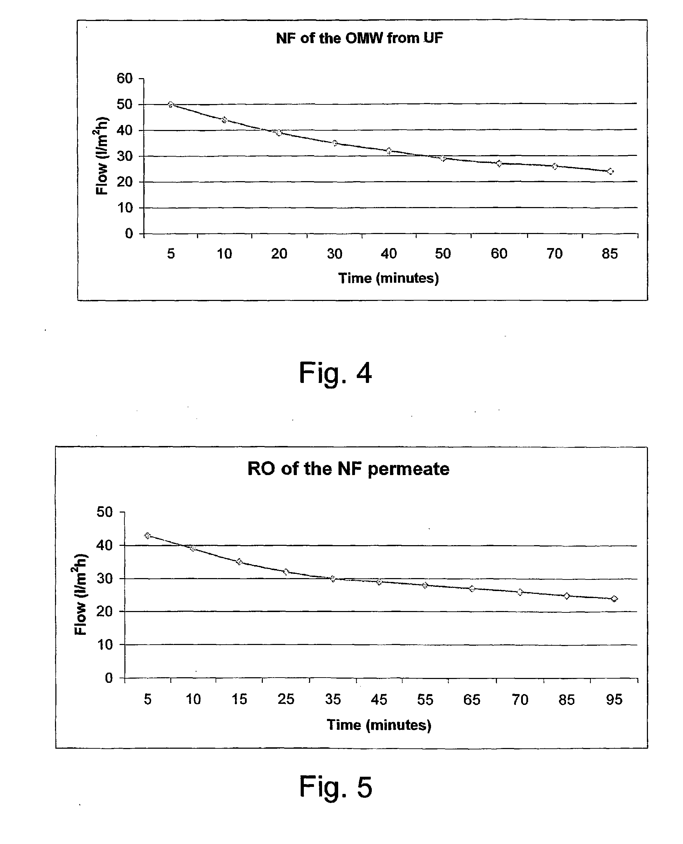

The diagram of Figure 4 shows the NF permeate flow trend over time (membrane productivity) at the operating conditions described.

### 8. The reverse osmosis operation

The previous NF permeate was subjected to reverse osmosis in order to concentrate all the polyphenol compounds contained. Starting from 119 l of NF permeate, reverse osmosis yields 12 l of concentrate containing concentrated polyphenol families, particularly hydroxytyrosol, and 107 l of permeate made up of ultrapure water.

The membrane used is spiral-shaped with dimensions of 4 x 40 inch, manufactured by Osmonics (USA), type AG 4040 F 1305, thin film polyamide, high saline rejection, with a surface of 6 m².

The process conditions are the following:
■ Feed flow rate: 2,000 l/h
■ Entry flow pressure: 30 bar
■ Exit flow pressure: 25 bar
■ Pressure on the permeate side: zero
■ Temperature: 15-25°C

During the trial, the average flow of permeate is around 25 l/m²h. The reverse osmosis plant contains a water-based heat-exchange system that stabilises the temperature between 20 and 30 °C.

At the end of the process, about 12 l of concentrate rich in polyphenols, particularly hydroxytyrosol, is collected. In the reverse osmosis concentrate, of a slightly yellow colour, the overall polyphenol concentration is around 30 g/l.

The reverse osmosis permeate is about 107 l and has a COD of 120 ppm of oxygen. It can be used as a liquid base for beverages, with the addition of saline integrators.

The diagram of Figure 5 shows the reverse osmosis permeate flow trend over time (membrane productivity) at the operating conditions described.

In the overall process described in the example, the polyphenols were concentrated with a factor of 10, but it must be considered that the MF and UF concentrates lose non-negligible amounts that the diafiltration recovers in part.

### 9. Membrane washing

After the filtration cycles, the membrane units were reconditioned to restore their initial flow conditions. Washing of the UF, NF and RO membranes did not require any particular procedures since the initial flow is quickly restored. These three units were washed in water in a totally recirculating network, about 20 l for 10 minutes. At the end, the water was drawn off and the membranes were washed again with a solution of 20 l of 0.05 M sodium hydroxide for 20 minutes. At the end of this wash, the units were rinsed with network water and then an acid wash was carried out using 0.5% nitric acid (20 l for 20 minutes).

At this point the membranes were rinsed in distilled water (RO permeate of the process).

The MF ceramic membranes required a more chemically aggressive wash, and the procedures were as follows:
■ Washing in network water, 30 l, for 15 minutes; washing in 20 l of 0.5 M sodium hydroxide at 80°C for 35 minutes; rinsing in network water (20 l) and subsequent washing in 25 l of 1.5% nitric acid at 80°C for 30 minutes. Finally, a rinse with distilled water (RO permeate) restores the initial flow conditions.

The above description shows how the present invention successfully meets the following aims:
■ making a raw substrate, which is particularly complex as regards chemical composition, filterable on a membrane;
■ selectively separating the microdispersed components (cellulose, hemicellulose and pectin) from the soluble ones (proteins, polyphenols, glucids, mineral salts);
■ recovering particularly the polyphenolic components for biomedical uses.
■ reusing the fractions separated with membranes for different uses (compost for agriculture and biogas);
■ rationalising water consumption in the overall olive milling process;
■ gaining revenues from the treatment of waste products of the olive oil milling process.

The present invention solves the serious problem of treating olive mill wastewater, reducing the problems of transporting huge volumes of liquids, with the consequent reduction in transportation costs and with evident environmental benefits, and namely avoiding the spreading of a phytotoxic industrial effluent over soils and the contamination of underground water sources.

The present invention has been disclosed with particular reference to some specific embodiments thereof, but it should be understood that modifications and changes may be made by the persons skilled in the art without departing from the scope of the invention as defined in the appended claims.

## Claims

1. A process for totally recovering the chemical components of olive mill wastewater (OMW) including, in sequence, the following operations:
a) collecting the raw OMW after the olive milling process;
b) adjusting the pH of the said OMW to within the range of 3-4.5;
c) treating the OMW obtained from the preceding operation by means of enzymatic hydrolysis;
d) separating out the particle and suspended components, resulting from the preceding operation, and a partially clarified liquid product;
e) treating the said liquid product resulting from operation d) by tangential microfiltration (MF), and providing a retentate phase and a permeate phase;
f) treating the permeate coming from the preceding operation by means of tangential nanofiltration (NF), and providing for a retentate phase and a permeate phase;
g) treating the permeate coming from operation f) by means of reverse osmosis (RO), and providing a retentate phase rich in purified polyphenols and a permeate composed of purified water;
the said solid phase resulting from operation d) and the said retentate phase resulting from operation e) being reusable as a substrate for compost production or for anaerobic fermentation processes, and the said retentate phase coming from operation f) being reusable for extracting the polyphenol compounds therefrom.

2. A process according to claim 1, wherein after the said operation e) of tangential microfiltration (MF) the following further operation is carried out:
e1) treating the said retentate phase obtained from MF by adding water and carrying out another microfiltration (diafiltration), with the production of a diafiltrate;
the liquid subjected to the subsequent operation f) of tangential nanofiltration being the sum of the permeate and of the diafiltrate coming from the preceding operations.

3. A process according to claim 2, wherein the permeate and diafiltrate coming from operations e) and e1) are subjected to the following operation:
e2)treatment by tangential ultrafiltration (UF) in order to obtain a retentate phase which can be reused as a substrate for compost production or for aerobic or anaerobic fermentation processes and a permeate which is fed into the next operation f) of nanofiltration.

4. A process according to any one of claims 1-3, wherein the said phase a) of OMW collection is carried out after less than 1-3 hours of OMW generation in the olive mill.

5. A process according to any one of claims 1-4, wherein in the said operation b) the pH of the OMW is adjusted to within the range of 3-4.5 by adding hydrogen chloride and citric acid.

6. The process according to any one of claims 1-5, wherein the said operation c) of enzymatic hydrolysis is carried out by adding a pectinase enzymatic complex to the acidified OMW.

7. A process according to any one of claims 1-6, wherein the said operation d) of separation is carried out by means of continuous centrifugation, with the production of a partially clarified liquid supernatant, which is subjected to the said subsequent operation e), and of a thick deposit that is wholly or partly fed back into the said operation c) of enzymatic hydrolysis and/or is reusable as a substrate for compost production or for anaerobic fermentation processes.

8. A process according to any one of claims 1-7, wherein the said operation e) of tangential microfiltration is carried out by means of ceramic membranes of molecular size ranging between 0.10 and 1.4 µm.

9. A process according to claim 8, wherein the said ceramic membranes for MF are made up of a ceramic block with 23 filtration channels in a daisy-like or sunflower-like arrangement.

10. The process according to claim 9, wherein the said ceramic membrane channels each have an average diameter of 3.4 mm and length of 1.2 m, with a filtering surface of 0.35 m² per ceramic block.

11. A process according to any one of claims 1-10, wherein downstream of each of the said operations e) of tangential microfiltration, e2) of tangential ultrafiltration, and f) of tangential nanofiltration a diafiltration is carried out by feeding the membrane with purified water obtained from the said operation g) of reverse osmosis, that is added to the retentate of MF.

12. A process according to any one of claims 1-11, wherein the said operation e1) of tangential ultrafiltration is carried out by means of polymeric membranes of molecular size ranging between 1 and 20 kDalton.

13. A process according to claim 12, wherein the said polymeric membranes for UF are spiral-shaped and made of one of the following materials: polysulphone, polyethersulphone polyamide or regenerated cellulose acetate.

14. A process according to claim 13, wherein the said spiral-shaped polymeric membranes for UF have filter channel sizes of 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

15. A process according to any one of claims 1-14, wherein downstream of the said operation e1) of tangential ultrafiltration a dialfiltration operation is carried out by feeding the membrane with purified water obtained from the said operation g) of reverse osmosis, which is added to the UF retentate.

16. A process according to each of claims 1-15, wherein the said operation f) of tangential nanofiltration is carried out by means of polymeric membranes of molecular size ranging between 150 and 250 Dalton.

17. A process according to claim 16, wherein the said polymeric membranes for NF are spiral-shaped and made of composite polyamide or nylon.

18. The process according to claim 17, wherein the said spiral-shaped polymeric membranes for NF have filter channel sizes of 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

19. A process according to any one of claims 1-18, wherein the said operation g) of reverse osmosis is carried out by means of high saline rejection polymeric membranes.

20. The process according to claim 19, wherein the said polymeric membranes for reverse osmosis are spiral-shaped and made of composite polyamide.

21. The process according to claim 20, wherein the said spiral-shaped polymeric membranes for reverse osmosis have filter channel sizes of 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

22. A process according to any one of the aforesaid claims, wherein the purified water product obtained as the permeate of the said operation g) of reverse osmosis has a COD lower than 100 ppm of O₂.

## Patentansprüche

1. Verfahren zur Gesamt-Rückgewinnung der chemischen Komponenten von Olivenmühlen-Abwasser (OMA), welches, nacheinander, die folgenden Vorgänge einschließt:
a) Gewinnen des rohen OMA nach dem Olivenmahlprozess;
b) Einstellen des pH-Werts dieses OMA innerhalb des Bereichs von 3-4,5;
c) Behandeln des aus dem vorhergehenden Vorgang erhaltenen OMA mittels enzymatischer Hydrolyse;
d) Trennen der Partikelkomponenten und suspendierten Komponenten, die das Ergebnis des vorhergehenden Vorgangs sind, und eines partiell geklärten flüssigen Produkts;
e) Behandeln des flüssigen Produkts, welches das Ergebnis des Vorgangs d) ist, durch Tangential-Mikrofiltration (MF) und Bereitstellen einer Retentat-Phase und einer Permeat-Phase;
f) Behandeln des aus dem vorhergehenden Vorgang stammenden Permeats mittels Tangential-Nanofiltration (NF) und Bereitstellen einer Retentat-Phase und einer Permeat-Phase;
g) Behandeln des aus dem Vorgang f) stammenden Permeats mittels reverser Osmose (RO) und Bereitstellen einer Retentat-Phase, die reich an gereinigten Polyphenolen ist, und eines Permeats, das aus gereinigtem Wasser besteht;
wobei die feste Phase, die aus dem Vorgang d) resultiert, und die Retentat-Phase, die aus dem Vorgang e) resultiert, als Substrat für die Kompostherstellung oder für anaerobe Fermentationsprozesse wiederverwendbar sind, und die Retentat-Phase, die aus dem Vorgang f) stammt, zur Extraktion der Polyphenol-Verbindungen daraus wiederverwendbar ist.

2. Verfahren nach Anspruch 1, wobei nach dem Vorgang e) der Tangential-Mikrofiltration (MF) der folgende weitere Vorgang durchgeführt wird:
e1) Behandeln der Retentat-Phase, die aus der MF erhalten wurde, durch Zugeben von Wasser und Durchführen einer weiteren Mikrofiltration (Diafiltration) unter Bildung eines Diafiltrats;
wobei die Flüssigkeit, die dem folgenden Vorgang f) der Tangential-Nanofiltration unterworfen wird, die Summe des Permeats und des Diafiltrats aus den vorhergehenden Vorgängen ist.

3. Verfahren nach Anspruch 2, wobei das Permeat und das Diafiltrat, die aus den Vorgängen e) und e1) stammen, dem folgenden Vorgang unterworfen werden:
e2) Behandlung mittels Tangential-Ultrafiltration (UF), um eine Retentat-Phase, welche als Substrat für die Kompostherstellung oder für aerobe oder anaerobe Fermentationsprozesse wiederverwendet werden kann, und ein Permeat, welches dem nächsten Vorgang f) der Nanofiltration zugeführt wird, zu erhalten.

4. Verfahren nach irgendeinem der Ansprüche 1-3, wobei die Phase a) der OMA-Gewinnung nach weniger als 1-3 Stunden nach der OMA-Erzeugung in der Olivenmühle durchgeführt wird.

5. Verfahren nach irgendeinem der Ansprüche 1-4, wobei in dem Vorgang b) der pH-Wert des OMA innerhalb des Bereichs von 3-4,5 durch Zugabe von Salzsäure und Citronensäure eingestellt wird.

6. Verfahren nach irgendeinem der Ansprüche 1-5, wobei der Vorgang c) der enzymatischen Hydrolyse durch Zugeben eines enzymatischen Pectinase-Komplexes zu dem angesäuerten OMA durchgeführt wird.

7. Verfahren nach irgendeinem der Ansprüche 1-6, wobei der Vorgang d) der Trennung durchgeführt wird durch kontinuierliche Zentrifugation unter Bildung eines partiell geklärten flüssigen Überstands, welcher dem nachfolgenden Vorgang e) unterworfen wird, und eines dicken Niederschlags, welcher ganz oder teilweise zu dem Vorgang c) der enzymatischen Hydrolyse rückgeführt wird und/oder als Substrat für Kompostherstellung oder für anaerobe Fermentationsprozesse wiederverwendbar ist.

8. Verfahren nach irgendeinem der Ansprüche 1-7, wobei der Vorgang e) der Tangential-Mikrofiltration mittels keramischer Membranen einer Molekulargröße im Bereich zwischen 0,10 und 1,4 µm durchgeführt wird.

9. Verfahren nach Anspruch 8, wobei die keramischen Membranen für die MF aus einem keramischen Block mit 23 Filtrationskanälen in einer Gänseblümchen-artigen oder Sonnenblumen-artigen Anordnung bestehen.

10. Verfahren nach Anspruch 9, wobei die keramischen Membrankanäle jeweils einen mittleren Durchmesser von 3,4 mm und eine Länge von 1,2 m, mit einer Filtrationsoberfläche von 0,35 m² pro Keramikblock, aufweisen.

11. Verfahren nach irgendeinem der Ansprüche 1-10, wobei stromabwärts eines jeden der Vorgänge e) der Tangential-Mikrofiltration, e2) der Tangential-Ultrafiltration und f) der Tangential-Nanofiltration eine Diafiltration durchgeführt wird, indem die Membran mit gereinigtem Wasser, erhalten aus dem Vorgang g) der reversen Osmose, beschickt wird, welches dem Retentat der MF zugegeben wird.

12. Verfahren nach irgendeinem der Ansprüche 1-11, wobei der Vorgang e1) der Tangential-Ultrafiltration mit polymeren Membranen einer Molekulargröße im Bereich von 1 bis 20 kDalton durchgeführt wird.

13. Verfahren nach Anspruch 12, wobei die polymeren Membranen für die UF spiralförmig sind und aus einem der folgenden Materialien hergestellt sind: Polysulfon, Polyethersulfon-Polyamid oder regeneriertes Celluloseacetat.

14. Verfahren nach Anspruch 13, wobei die spiralförmigen polymeren Membranen für die UF Filterkanalgrößen von 4 Zoll Durchmesser x 40 Zoll Länge (10,16 cm x 101,6 cm), 6 Zoll Durchmesser x 40 Zoll Länge (15,24 cm x 101,6 cm) oder 8 Zoll Durchmesser x 40 Zoll Länge (20,32 cm x 101,6 cm) aufweisen.

15. Verfahren nach irgendeinem der Ansprüche 1-14, wobei stromabwärts des Vorgangs e1) der Tangential-Ultrafiltration ein Diafiltrations-Vorgang durchgeführt wird, indem die Membran mit gereinigtem Wasser, erhalten aus dem Vorgang g) der reversen Osmose, beschickt wird, welches dem UF-Retentat zugegeben wird.

16. Verfahren nach jedem der Ansprüche 1-15, wobei der Vorgang f) der Tangential-Nanofiltration mit polymeren Membranen einer Molekulargröße im Bereich zwischen 150 und 250 Dalton durchgeführt wird.

17. Verfahren nach Anspruch 16, wobei die polymeren Membranen für die NF spiralförmig sind und aus Verbund-Polyamid oder Nylon bestehen.

18. Verfahren nach Anspruch 17, wobei die spiralförmigen polymeren Membranen für die NF Filterkanalgrößen von 4 Zoll Durchmesser x 40 Zoll Länge (10,16 cm x 101,6 cm), 6 Zoll Durchmesser x 40 Zoll Länge (15,24 cm x 101,6 cm) oder 8 Zoll Durchmesser x 40 Zoll Länge (20,32 cm x 101,6 cm) aufweisen.

19. Verfahren nach irgendeinem der Ansprüche 1-18, wobei der Vorgang g) der reversen Osmose mittels polymerer Membranen mit hoher Salzabweisung durchgeführt wird.

20. Verfahren nach Anspruch 19, wobei die polymeren Membranen für die reverse Osmose spiralförmig sind und aus Verbund-Polyamid bestehen.

21. Verfahren nach Anspruch 20, wobei die spiralförmigen polymeren Membranen für die reverse Osmose Filterkanalgrößen von 4 Zoll Durchmesser x 40 Zoll Länge (10,16 cm x 101,6 cm), 6 Zoll Durchmesser x 40 Zoll Länge (15,24 cm x 101,6 cm) oder 8 Zoll Durchmesser x 40 Zoll Länge (20,32 cm x 101,6 cm) aufweisen.

22. Verfahren nach irgendeinem der vorhergehenden Ansprüche, wobei das gereinigte Wasserprodukt, das als Permeat des Vorgangs g) der reversen Osmose erhalten wird, einen COD-Wert von weniger als 100 ppm an O₂ aufweist.

## Revendications

1. Procédé pour la récupération totale des composants chimiques des effluents d'huileries d'olive, comprenant successivement les opérations suivantes :
a) collecte des effluents d'huileries d'olive bruts après l'opération de trituration des olives ;
b) ajustement du pH desdits effluents d'huileries d'olive à une valeur comprise dans la plage de 3 à 4,5 ;
c) traitement des effluents d'huileries d'olive obtenus de l'opération précédente, au moyen d'une hydrolyse enzymatique ;
d) séparation des composants particulaires et des composants en suspension, résultant de l'opération précédente, et d'un produit liquide partiellement clarifié ;
e) traitement dudit produit liquide résultant de l'opération d) par microfiltration (MF) tangentielle, et obtention d'une phase rétentat et d'une phase perméat ;
f) traitement du perméat provenant de l'opération précédente au moyen d'une nanofiltration (NF) tangentielle, et obtention d'une phase rétentat et d'une phase perméat ;
g) traitement du perméat provenant de l'opération f) au moyen d'une osmose inverse, et obtention d'une phase rétentat riche en polyphénols purifiés et d'un perméat composé d'eau purifiée ;
ladite phase solide résultant de l'opération d) et ladite phase rétentat provenant de l'opération e) pouvant être réutilisées en tant que substrat pour la production d'un compost ou pour des processus de fermentation anaérobie, et ladite phase rétentat provenant de l'opération f) pouvant être réutilisée pour en extraire les composés polyphénols.

2. Procédé selon la revendication 1, dans lequel, après l'opération e) de microfiltration (MF) tangentielle, on met en oeuvre l'opération supplémentaire suivante :
e1) traitement de ladite phase rétentat obtenue par MF, par addition d'eau et mise en oeuvre d'une autre microfiltration (diafiltration), avec production d'un diafiltrat ;
le liquide soumis à l'opération ultérieure f) de nanofiltration tangentielle étant la somme du perméat et du diafiltrat provenant des opérations précédentes.

3. Procédé selon la revendication 2, dans lequel le perméat et le diafiltrat provenant des opérations e) et e1) sont soumis aux opérations suivantes :
e2) traitement par ultrafiltration (UF) tangentielle dans le but d'obtenir une phase rétentat qui peut être réutilisée en tant que substrat pour la production d'un compost, ou pour des processus de fermentation aérobie ou anaérobie,
et un perméat qui est utilisé comme charge dans l'opération suivante f) de nanofiltration.

4. Procédé selon l'une quelconque des revendications 1 à 3, dans lequel ladite phase a) de collecte des effluents d'huileries d'olive est mise en oeuvre après moins de 1 à 3 heures de génération d'effluents d'huileries d'olive dans l'huilerie.

5. Procédé selon l'une quelconque des revendications 1 à 4, dans lequel, dans ladite opération b), le pH des effluents d'huileries d'olive est ajusté à une valeur comprise dans la plage de 3 à 4,5 par addition de chlorure d'hydrogène et d'acide citrique.

6. Procédé selon l'une quelconque des revendications 1 à 5, dans lequel ladite opération c) d'hydrolyse enzymatique est mise en oeuvre par addition d'un complexe enzymatique de pectinase aux effluents d'huileries d'olive acidifiés.

7. Procédé selon l'une quelconque des revendications 1 à 6, dans lequel ladite opération d) de séparation est mise en oeuvre au moyen d'une centrifugation continue, avec production d'un surnageant liquide partiellement clarifié, qui est soumis à ladite opération ultérieure e), et d'un dépôt épais, qui en totalité ou en partie est renvoyé en tant que charge dans ladite opération c) d'hydrolyse enzymatique et/ou est réutilisable en tant que substrat pour la production d'un compost ou pour des processus de fermentation anaérobie.

8. Procédé selon l'une quelconque des revendications 1 à 7, dans lequel ladite opération e) de microfiltration tangentielle est mise en oeuvre au moyen de membranes céramiques ayant une taille moléculaire comprise dans la plage de 0,10 à 1,4 µm.

9. Procédé selon la revendication 8, dans lequel lesdites membranes céramiques pour MF sont constituées d'un bloc céramique comportant 23 canaux de filtration, selon une disposition en marguerite ou en tournesol.

10. Procédé selon la revendication 9, dans lequel lesdits canaux des membranes céramiques ont chacun un diamètre moyen de 3,4 mm et une longueur de 1,2 m, avec une surface de filtration de 0,35 m² par bloc céramique.

11. Procédé selon l'une quelconque des revendications 1 à 10, dans lequel, en aval de chacune desdites opérations e) de microfiltration tangentielle, e2) d'ultrafiltration tangentielle et f) de nanofiltration tangentielle, on met en oeuvre une diafiltration en alimentant la membrane avec une eau purifiée obtenue à partir de ladite opération g) d'osmose inverse, qui est ajoutée au rétentat de l'opération de MF.

12. Procédé selon l'une quelconque des revendications 1 à 11, dans lequel ladite opération e1 d'ultrafiltration tangentielle est mise en oeuvre au moyen de membranes polymères ayant une taille moléculaire comprise dans la plage de 1 à 20 kDa.

13. Procédé selon la revendication 12, dans lequel lesdites membranes polymères pour UF ont une forme de spirale et sont constituées de l'un des matériaux suivants : polysulfone, polyéthersulfone, polyamide ou acétate de cellulose régénérée.

14. Procédé selon la revendication 13, dans lequel lesdites membranes polymères en forme de spirale pour UF ont des tailles de canaux de filtration correspondant à un diamètre de 4 pouces et à une longueur de 40 pouces (10,16 cm x 101,6 cm), à un diamètre de 6 pouces et à une longueur de 40 pouces (15,24 cm x 101,6 cm), ou à un diamètre de 8 pouces et à une longueur de 40 pouces (20,32 cm x 101,6 cm).

15. Procédé selon l'une quelconque des revendications 1 à 14, dans lequel, en aval de ladite opération e1) d'ultrafiltration tangentielle, on procède à une opération de diafiltration en alimentant la membrane avec de l'eau purifiée obtenue de ladite opération g) d'osmose inverse, qui est ajoutée au rétentat de l'opération UF.

16. Procédé selon chacune des revendications 1 à 15, dans lequel ladite opération f) de nanofiltration tangentielle est mise en oeuvre au moyen de membranes polymères ayant une taille moléculaire comprise dans la plage de 150 à 250 Da.

17. Procédé selon la revendication 16, dans lequel lesdites membranes polymères pour NF ont une forme spirale et sont constituées de polyamide composite ou de nylon.

18. Procédé selon la revendication 17, dans lequel lesdites membranes polymères en forme de spirale pour NF ont des tailles de canaux de filtration correspondant à un diamètre de 4 pouces et à une longueur de 40 pouces (10,16 cm x 101,6 cm), à un diamètre de 6 pouces et à une longueur de 40 pouces (15,24 cm x 101,6 cm) ou à un diamètre de 8 pouces et à une longueur de 40 pouces (20,32 cm x 101,6 cm).

19. Procédé selon l'une quelconque des revendications 1 à 18, dans lequel ladite opération g) d'osmose inverse est mise en oeuvre au moyen de membranes polymères à fort taux de rejet de solution saline.

20. Procédé selon la revendication 19, dans lequel lesdites membranes polymères pour osmose inverse ont une forme de spirale et sont constituées de polyamide composite.

21. Procédé selon la revendication 20, dans lequel lesdites membranes polymères en forme de spirale pour osmose inverse ont des tailles de canaux de filtration correspondant à un diamètre de 4 pouces et à une longueur de 40 pouces (10,16 cm x 101,6 cm), à un diamètre de 6 pouces et à une longueur de 40 pouces (15,24 cm x 101,6 cm) ou à un diamètre de 8 pouces et à une longueur de 40 pouces (20,32 cm x 101,6 cm).

22. Procédé selon l'une quelconque des revendications précédentes, dans lequel le produit eau purifiée obtenue en tant que perméat de ladite opération g) d'osmose inverse a une DCO inférieure à 100 ppm d'O₂.
